(19) **Europäisches Patentamt**

**European Patent Office**

**Office européen des brevets**

(11) **EP 0 776 317 B1**

(12) **EUROPEAN PATENT SPECIFICATION**

(45) Date of publication and mention
of the grant of the patent:
**03.11.1999 Bulletin 1999/44**

(21) Application number: **95907323.0**

(22) Date of filing: **06.01.1995**

(51) Int Cl.$^6$: **C01F 7/00**, C01B 13/36

(86) International application number:
**PCT/US95/00166**

(87) International publication number:
**WO 96/05140 (22.02.1996 Gazette 1996/09)**

(54) **TWO POWDER SYNTHESIS OF HYDROTALCITE AND HYDROTALCITE-LIKE COMPOUNDS**

ZWEI-PULVER-VERFAHREN ZUR HERSTELLUNG VON HYDROTALCIT UND
HYDROTALCITÄHNLICHEN VERBINDUNGEN

SYNTHESE D'HYDROTALCITE ET DE COMPOSES APPARENTES, A PARTIR DE DEUX
POUDRES

(84) Designated Contracting States:
**AT BE DE DK ES FR GB GR IE IT NL PT SE**

(30) Priority: **15.08.1994 US 290220**

(43) Date of publication of application:
**04.06.1997 Bulletin 1997/23**

(73) Proprietor: **ALUMINUM COMPANY OF AMERICA**
**Alcoa Center, Pennsylvania 15069-0001 (US)**

(72) Inventors:
• **MARTIN, Edward, S.**
  **Alcoa Center, PA 15069-0001 (US)**
• **HORN, William, E., Jr.**
  **Alcoa Center, PA 15069-0001 (US)**
• **STINSON, John, M.**
  **Alcoa Center, PA 15069-0001 (US)**
• **CEDRO, Vito, III**
  **Alcoa Center, PA 15069-0001 (US)**

(74) Representative: **Baillie, Iain Cameron**
**Ladas & Parry,**
**Dachauerstrasse 37**
**80335 München (DE)**

(56) References cited:
**EP-A- 0 536 879**      **DE-A- 3 346 943**
**FR-A- 2 417 472**      **US-A- 3 650 704**
**US-E- R E34 164**

• **CLAY AND CLAY MINERALS, vol.34, no.5, 1986**
  **pages 507 - 510 I. PAUSCH ET AL. 'Syntheses of**
  **disordered and Al-rich hydrotalcite-like**
  **compounds.'**
• **MINERALOGICAL MAGAZINE, vol.43, 1980 G.**
  **MASCOLO ET AL. 'A new synthesis and**
  **characterization of magnesium-aluminium**
  **hydroxides.'**

## Description

This invention relates to the field of making mixed metal hydroxides or layered double hydroxide products. More specifically, the invention relates-to an improved two-step method for making hydrotalcite and hydrotalcite-like compounds from dry powder constituents.

Hydrotalcite exists in both a natural and synthetic form. Naturally occurring deposits have been found in Snarum, Norway and in the Ural Mountains. Typical occurrences are in the form of serpentines, talc schists, or as an alteration product where hydrotalcite forms the pseudomorph of a spinel. Like most ores, natural hydrotalcite is virtually impossible to find in a pure state. Such deposits often contain one or more other minerals including penninite and muscovite.

Several methods are known for making synthetic hydrotalcite in such product forms as a fine powder, -20 mesh granules or as 0·3175 cm (1/8-inch) diameter extrudates. One representative method is described in U.S. Patent No. 3,539,306. There, an aluminum hydroxide, aluminum-amino acid salt, aluminum alcoholate, water soluble aluminate, aluminum nitrate and/or aluminum sulfate are mixed with a magnesium component selected from magnesium oxide, magnesium hydroxide or water-soluble magnesium salt and a carbonate ion-containing compound in an aqueous medium maintained at a pH of 8 or more. The resulting product may be used as a stomach antacid. In this typical neutralization process, a fairly pure, finely sized hydrotalcite particle is formed. A serious disadvantage of this method, however, is its formation of a sodium salt by-product. This salt neutralization process for making hydrotalcites could also produce a brucite-like structure with undesired anions (e.g. sulfate) or cations (Na$^+$) included therein.

In Misra Reissue U.S. Patent No. 34,164, yet another means for synthesizing hydrotalcite is taught. The method comprises heating magnesium carbonate and/or magnesium hydroxide to form activated magnesia, then combining the activated magnesia with an aqueous solution of aluminate, carbonate and hydroxyl ions.

Other known methods for synthesizing hydrotalcite include: adding dry ice or ammonium carbonate to a thermal decomposition product from a magnesium nitrate-aluminum nitrate mixture, after which intermediate product is subjected to temperatures below about 163°C (325°F). and pressures of 13·8 to 137·9 MPa (2,000 to 20,000 psi). Yet another process, from "Properties of a Synthetic Magnesium-Aluminum Carbonate Hydroxide and its Relationship to Magnesium-Aluminum Double Hydroxide Manasseite, and Hydrotalcite", The American Mineralogist, Vol. 52, pp. 1036-1047 (1967), produces hydrotalcite-like materials by titrating a solution of MgCl$_2$ and AlCl$_3$ with NaOH in a carbon dioxide-free system. This suspension is dialyzed for 30 days at 60°C. to form a hydrated Mg-Al carbonate hydroxide having the properties of both manasseite and hydrotalcite.

In Clay and Clay Minerals, volume 34, number 5, pages 507-10 (1986), I. Pausch et al. disclose a method for making hydrotalcite-like compounds, but they do not disclose a method for using aluminum oxide to make meixnerite. EP-A-536879 (Amoco) discloses a method for making hydrotalcite-type clays using aluminum salts, but not aluminum oxide.

It is a principal objective of this invention to provide an improved means for making synthetic hydrotalcite and hydrotalcite-like compounds from two or more relatively inexpensive, dry powder components. It is another objective to provide an improved process for making hydrotalcite and related materials with less sodium ion contamination. It is still another objective to provide a method for synthesizing hydrotalcite without depending on the use of any alumina gels. It is still another objective to make hydrotalcite and hydrotalcite-like compounds through the further processing of an improved meixnerite product, itself made by combining activated magnesia with a high surface area, transition alumina.

Yet another principal objective is to make hydrotalcite and hydrotalcite-like compounds in a more environmentally acceptable manner. According to preferred embodiments, the synthetic hydrotalcites made by the methods described hereinbelow yield no by-products other than water. Any remaining discharge waters should be easily disposable due to their low dissolved solids content.

These objectives are met by the method for making a layered double hydroxide powder which comprises: (a) reacting at least one trivalent metal oxide powder and at least one divalent metal compound selected from the group consisting of a hydroxide, oxide, carbonate and mixtures thereof, in an aqueous suspension to form a double hydroxide intermediate; (b) contacting the double hydroxide intermediate with an anion source to form a layered double hydroxide; and (c) separating the layered double hydroxide from the suspension. The method comprises reacting powdered magnesium oxide with a high surface area, transition alumina in a suspension or slurry to form meixnerite or a meixnerite-like intermediate. The latter intermediate is then contacted with an anion source such as an acid or acid precursor, most preferably carbon dioxide, to form the layered double hydroxide compound which is separated from the suspension by filtering, centrifugation, vacuum dehydration or other known means. On a preferred basis, the transition alumina so combined with activated magnesia consists essentially of an activated alumina powder having a surface area of about 100 m$^2$/g or greater. For related double hydroxide formations, still other reactants, such as bromides, chlorides, boric acids, or their salts, are combined with the meixnerite intermediate to make similarly structured, brucite-like layered double hydroxide family members.

## 1. Definitions

As used herein, the following terms shall have the meanings provided hereinbelow:

a. "Transition alumina" means a high surface area alumina in a powdered or fine particulate form. One preferred way of defining such alumina materials uses surface area and Loss on Ignition (LOI) measurements. More specifically, an alumina having a Brunauer-Emmett-Teller [or B.E.T.] measured surface area of about 100 $m^2/g$ or more would be considered as having a high surface area and thus qualify as a transition alumina for purposes of this invention. Aluminas having an LOI weight percentage of about 1.5% or more would also qualify under this definition.

One particular preferred type of transition aluminas is referred to as a "rehydratable alumina". It tends to form strong hydroxyl bonds on contact with water and its rehydration reactions are highly exothermic. The average particle sizes for such aluminas may range from 0.01-200 $\mu$, with a range of 0.1 to 10 or 20 micrometers being more preferred.

Certain activated aluminas are more suitable than others for purposes of this invention. Most are high surface area aluminas formed by the rapid calcination of hydrated alumina at temperatures below that required for complete dehydration or calcination. Typically, such aluminas are amorphous (i.e., have no microcrystalline structure) as determined by X-ray diffraction. These powders exhibit an LOI value of 4-12% by weight, and a BET surface area of 200-300 $m^2/g$.

b. "Activated magnesia" or activated magnesium oxide refers to the magnesium-based product activated by "soft burning" MgO at one or more temperatures between 450 and 900°C. This component has a general surface area of 10-200 $m^2/g$, preferably 20-150 $m^2/g$ and an L.O.I. ranging from 1.0 to 6.0 wt.%. Such criteria distinguishes this reactant from magnesias which have been dead-burned or completely calcined. Although the latter may still produce meixnerite with longer reaction times or under more strenuous reaction conditions, the percent yields from such conditions are significantly lower than those preferred for the present invention.

There are numerous means for making an activated magnesia product to combine with transition aluminas according to the first method step of this invention. For example, commercially sold magnesium carbonate can be heated to drive off carbon dioxide and thus form a reactive magnesia thereby. Magnesium oxide may also be made by: (a) heating natural or synthetic magnesium hydroxides or basic magnesium carbonate, to temperatures between 380 and 950°C.; or (b) by heating $MgCl_2$ with lime. Various known methods-may be used to generate magnesia powders of various particles sizes and/or surface areas.

c. "Hydrotalcite" compounds shall be understood to apply to the structural family of layered double hydroxides whose family members consist of any compound having the formula: $A_wB_x(OH)_yC_z \cdot nH_2O$, wherein A represents a divalent metal cation, B a trivalent metal cation, C a mono- to polyvalent anion, and w, x, y, z and n satisfy the following conditions: $0 < z \leq x \leq 4 \leq w \leq 1/2y$ and $12 \geq n \geq 1/2(w-x)$. Preferred embodiments of this family have been identified by the formula: $A_6B_2(OH)_{16}C_z \cdot 4H_2O$, wherein A is selected from: $Mg^{2+}$, $Ni^{2+}$, $Cu^{2+}$, $Ca^{2+}$, $Fe^{2+}$ and $Zn^{2+}$; B from: $Al^{3+}$, $Fe^{3+}$ and $Cr^{3+}$; and C from an anion list which includes: $OH^-$, $Cl^-$, $Br^-$, $NO_3^-$, $CH_3COO^-$, $CO_3^{2-}$, $SO_4^{2-}$, $PO_4^{3-}$, $Fe(CN)_6^{3-}$, $Fe(CN)_6^{4-}$ and some borates, carboxylates and polyoxometallates, with $1/2 \leq z \leq 2$ (depending on the charge of the anion substituted therein). Some references refer to any compound having the aforementioned formulae as "hydrotalcite". For purposes of this invention, however, this family of structural compounds has been divided into various subgroups depending on the divalent and trivalent cations within its alternating brucite-like layers. For example, pyroaurites have the basic formula: $Mg_6Fe_2(OH)_{16}CO_3 \cdot 4H_2O$. Such compounds are also known as "sjogrenites". Collectively, these other family members have been referred to as "hydrotalcite-like" compounds.

Yet another preferred definition for the term "hydrotalcite" includes any natural or synthetic compound satisfying the formulae: $Mg_6Al_2(OH)_{16}CO_3 \cdot 4H_2O$ or $Mg_4 Al_2(OH)_{12} CO_3 \cdot 3H_2O$. This compound has sometimes been written as: $6MgO \cdot Al_2O_3 \cdot CO_2 \cdot 12H_2O$. In its ionic form, hydrotalcite may appear as: $[Mg_6Al_2(OH)_{16}]^{2+} \cdot [CO_3]^{2-} \cdot 4H_2O$. The main structural unit for this compound is brucite, or magnesium hydroxide $(Mg(OH)_2)$ having the form of an octagonal sheet with Mg ions positioned between multiple (OH) ions which share adjacent edges. By substituting trivalent aluminum ions for some of the divalent magnesium of this structure, sublayers of magnesium and aluminum are created while still maintaining brucite's basic sheet-like structure. To compensate for the charge imbalance from these aluminum ion substitutions, anions (indicated by letter "C" in the foregoing formulae) and water molecules are intercalated therein to form interlayers of $(C_z \cdot nH_2O)$ between the brucite-like structural layers, with $1/2 \leq z \leq 2$ depending on the anion so intercalated. The anion having the greatest affinity to combine with water in this structure and form hydrotalcite is carbonate $(CO_3^{2-})$. Sulfate $(SO_4^{2-})$ is another compatible anion.

The spacial distribution of carbonate ions within hydrotalcite partially depends on how the $Al^{3+}$ ions substitute for the $Mg^{2+}$ ions therein. Brucite layer spacing is also a function of the amount or degree of aluminum substitution into hydrotalcite's basic structure. As aluminum substitution increases, interlayer spacing decreases due to an increase in the electrostatic attraction between positive hydroxide layers and hydrotalcite's negative interlayers. Interlayer thicknesses may also vary depending on the size and orientation of the anions substituted for some'or all of the carbonate ions in hydrotalcite. From preferred embodiments, a hydrotalcite material having a Mg:Al ratio of 2 (x = 0.33) to 3 (x = 0.25) or higher is contemplated.

d. "Basic magnesium carbonate" means a dimagnesium salt containing hydroxide and carbonate anions in the same powder product, sometimes represented by the formula $Mg(OH)_2 \bullet MgCO_3$.

e. "Meixnerite" means a hydrotalcite-like, layered double hydroxide material in which all the intercalated anions are hydroxyls.

One generic means for summarizing the reactions believed to occur by the method described herein is as follows:

$$\text{Step 1. } M_a O_b + Al_2O_3 \bullet gH_2O \Rightarrow MX$$

$$\text{Step 2. } MX + HA \Rightarrow \text{Layered Double Hydroxide} + H_2O.$$

On a more preferred basis, the following two steps are believed to occur for hydrotalcite manufacture:

Step 1:

$$(1 - x)MgO + x/2\, Al_2O_3 + (1 + x/2 + w)H_2O \Rightarrow$$

$$[Mg_{(1-x)}Al_x(OH)_2]\,(OH)_x \bullet w\, H_2O$$

followed by Step 2:

$$2[Mg_{(1-x)}Al_x(OH)_2]\,(OH)_x \bullet w\, H_2O + x/2CO_2 \Rightarrow$$

$$2[Mg_{(1-x)}Al_x(OH)_2](CO_3{}^{2-})_{x/2} \bullet w\, H_2O + x\, H_2O$$

For some dry powder reactants, temperature limitations on the contacting water solution have proven beneficial to overall yield. While Step 1 of the foregoing reaction may proceed at temperatures as low as 25°C. for calcium-containing compounds, they usually proceed best at one or more temperatures between 80° and 160°C., especially for magnesium-containing, layered double hydroxides made by the method of this invention. At such temperatures, yields in excess of about 75% are commonly observed. More preferred reaction temperatures generally run between about 98 and 150°C. Though higher reaction pressures, up to about 0·81 MPa (8 atmospheres (or atm)), have been known to enhance the synthesis of hydrotalcite and hydrotalcite-like compounds according to this invention, more preferred reaction pressures are usually between ambient and 0·48 MPa (4.7 atms), as determined by the vapor pressure of water.

Suitable end uses for the hydrotalcite products made by this method include acid neutralizers and scavengers, especially for polypropylene and polyethylene manufacturers, adsorbents for heavy metal anions from waste waters, stabilizing components for other polymer systems such as poly (vinyl chloride), flame retarders, smoke suppressers, catalysts, catalyst supports and viscosity control agents.

Further features, objects and advantages of the present invention will be made clearer from the detailed description of examples which follows. It is to be understood, however, that such examples are merely representative of this invention and should not be used to limit its scope in any manner.

EXAMPLES 1 - 7

Each of the following were conducted using a 1.8 liter capacity, internally stirred reactor charged with 750 ml of deionized water. In each case, after the respective divalent and trivalent metal compounds were added to the water and dispersed therethrough with continuous stirring, carbon dioxide was bubbled into the reactor from a pressurized cylinder. When respective reaction times were completed, the reactor was allowed to cool and excess carbon dioxide gradually vented into the atmosphere. The resulting slurry was then vacuum filtered using a Buchner funnel and a sample of each filtrate was further dried under vacuum before x-ray diffraction analyses were conducted thereon to determine which crystal phases were present in these dried solids.

COMPARATIVE EXAMPLE 1

100 grams of hydromagnesite having the formula $Mg_5(CO_3)_4(OH)_2 \bullet 4H_2O$ and 47 grams of ground aluminum hydroxide having an average particle size of 10.0 μm were charged to the reactor. Carbon dioxide was added until the reactor pressure reached 3·47 MPa (34.3 atm). The reactor temperature was then maintained between 25-26°C. for about 4 hours. Analysis of the dried solids removed from this reaction showed the presence of hydromagnesite and alumina as gibbsite but no hydrotalcite.

COMPARATIVE EXAMPLE 2

For this example, another 100 grams of hydromagnesite were charged with 41.7 grams of the same ground $Al(OH)_3$ as in Example 1. Liquid carbon dioxide was added until the reactor pressure reached 3·69 MPa (36.4 atm). The reactor temperature was then maintained between 48-53°C. for about 4 hours. Analysis of the dried solids removed from this reaction again showed the presence of hydromagnesite and gibbsite but no hydrotalcite.

COMPARATIVE EXAMPLE 3

The same quantity of hydromagnesite and ground Al(OH)$_3$ used for Example 2 were again charged to a reactor for this Example. With 4·45 MPa (43.9 atm) of carbon dioxide added, the reactor charged for 4 hours at 90°C. still showed no sign of hydrotalcite in the recovered solids.

COMPARATIVE EXAMPLE 4

For this Example, 100 grams of the same hydromagnesite as before were charged with 31.0 grams of a rehydratable alumina having an average particle size of 2.0 μm. The slurry was stirred at room temperature for 3 hours while enough liquid carbon dioxide was added to raise the overall reactor pressure to 4·06 MPa (40.1 atm). The whole system was then heated to 50°C. for 2 hours. The dried filter cake from this reaction was found to contain major amounts of hydrotalcite by x-ray diffraction analysis.

COMPARATIVE EXAMPLE 5

For this Example, 100 grams of the same hydromagnesite as before were charged with 38.7 grams of a pseudoboehmite sold by Vista Chemical Co. under the tradename Catapal® SB, said material consisting of 65 μm diameter agglomerates of 0.1 μm basic particles. Enough carbon dioxide was added to take overall reactor pressure to 4·31 MPa (42.5 atm). The system was then kept between 48-52°C. for 4 hours. X-ray diffraction analysis of the resulting filter cake showed that major amounts of hydrotalcite were present.

COMPARATIVE EXAMPLE 6

The same quantities of hydromagnesite and pseudoboehmite used for Example 5 were again charged to a reactor for this Example. With 5·27 MPa (52.0 atm) of carbon dioxide added, the reactor charged for 4 hours at 90°C. resulted in a filter cake which had major amounts of hydrotalcite present (by x-ray diffraction analysis).

COMPARATIVE EXAMPLE 7

The same quantities of hydromagnesite and rehydratable alumina used for Example 4 were again charged to a reactor, but for this Example no additional carbon dioxide was added thereto. The system was heated to 50°C. for 2 hours. The resulting filter cake was analyzed to contain major amounts of hydrotalcite as well. However, the degree of hydromagnesite conversion for Example 7 was less than in Example 4 based on a comparison of x-ray diffraction peak intensities for these products.

EXAMPLES 8 - 13

For each of these examples, about 70 grams of MgO and 45.6 grams of rehydratable Al$_2$O$_3$ were mixed with 1200 ml of deionized water in a round-bottom flask to form a slurry. The slurry was then stirred and heated to atmospheric boiling. The area in the flask over the slurry was purged with nitrogen to prevent reaction with $CO_2$ from the air. After six (6) hours in the reactor, samples were removed and analyzed. Considerable meixnerite was found in these samples. After 22 hours at boiling, conversion was nearly complete. Several portions of this slurry were then cooled below 40°C. and treated with carbon dioxide gas or atmospheric air for converting the meixnerite to hydrotalcite. Samples removed from this slurry were analyzed and shown to contain major amounts of hydrotalcite. For Example 11, an oxalate was formed by adding oxalic acid to the meixnerite slurry at 26°-30°C. A borate form of hydrotalcite was made by adding boric acid to the meixnerite slurry for Example 12 and a stearate form was made by contacting meixnerite with stearic acid per Example 13.

**Claims**

1. A method for making a layered double hydroxide powder comprises:

    (a) reacting at least one trivalent metal oxide powder and at least one divalent metal compound selected from the group consisting of a hydroxide, oxide, carbonate and mixtures thereof, in an aqueous suspension to form a double hydroxide intermediate;
    (b) contacting the double hydroxide intermediate with an anion source to form a layered double hydroxide; and
    (c) separating the layered double hydroxide from the suspension.

2. The method of claim 1, wherein (1) the trivalent metal oxide powder is a compound consisting essentially of transition alumina; and/or (2) the divalent metal compound is selected from the group consisting of a magnesium oxide, hydroxide, carbonate or mixture thereof, a zinc oxide-containing compound, a copper oxide-containing compound, a nickel oxide-containing compound, an iron oxide-containing compound, a calcium oxide-containing compound, or a manganese oxide-containing compound or mixture thereof.

3. The method of claim 1 or 2, wherein (1) the trivalent metal oxide powder consists essentially of a rehydratable alumina powder, preferably an activated alumina having a BET surface area of about 100 m$^2$/g or greater; and/or (2) the divalent metal compound is selected from the group consisting of a magnesium oxide, hydroxide, carbonate or mixture thereof, and preferably is basic magnesium carbonate, magnesium oxide, hydromagnesite or mixture thereof, preferably hydromagnesite.

4. The method of claim 1, 2 or 3, wherein the anion source in step (b) is selected from the group consisting of: carbon dioxide; a carbonate-containing compound; an acid selected from the group consisting of: hydrogen bromide, boric acid, acetic acid, oxalic acid, stearic acid; and an ammonium salt of an acid selected from the group consisting of: hydrogen bromide, boric acid, acetic acid, oxalic acid, stearic acid and mixtures thereof.

5. A method according to any one of claims 1 to 4, which comprises:

   (a) reacting a magnesium-containing powder and a transition alumina powder in an aqueous suspension to form a meixnerite intermediate;
   (b) contacting the meixnerite intermediate with a carbonate containing ion, preferably carbon dioxide, to form a hydrotalcite compound; and
   (c) separating the hydrotalcite compound from the suspension.

6. A method according to any one of claims 1 to 3, which comprises:

   (a) reacting a magnesium-containing powder and a transition alumina powder in an aqueous suspension to form a meixnerite intermediate;
   (b) contacting the meixnerite intermediate with an alkali metal salt is selected from the group consisting of a chloride, a bromide, or an iodide or mixture thereof; and
   (c) separating the hydrotalcite-like compound from the suspension.

7. The method of claim 6, wherein the alkali metal salt is sodium bromide for making a brominated hydrotalcite-like compound in step (c) or is sodium chloride for making a chlorinated hydrotalcite-like compound in step (c); and/or step (a) is performed at one or more temperatures above 80°C, preferably 98°-150°C.

8. A method according to any one of claims I to 3, which comprises:

   (a) reacting a magnesium-containing powder and a transition alumina powder in an aqueous suspension to form a meixnerite intermediate;
   (b) contacting the meixnerite intermediate with an excess of borate ions to form a boron-containing, hydrotalcite-like compound; and

   (c) separating the boron-containing, hydrotalcite-like compound from the suspension.

9. The method of claim 8, wherein the borate ion contacting step (b) comprises adding to the suspension a boron compound which is selected from the group consisting of $H_3BO_3$, $H_3B_3O_6$, $Na_2B_2O_7 \cdot 10H_2O$, $Na_2B_2O_7$, $NaBO_2NaB_5O_8$, or $K_2B_{10}O_{16}$ or mixtures thereof.

10. A method according to any one of claims 1 to 3, which comprises:

    (a) reacting a magnesium-containing powder and a transition alumina powder in an aqueous suspension to form a meixnerite intermediate;
    (b) contacting the mcixnerite intermediate with an excess of oxalate ions to form an oxalate-containing, hydrotalcite-like compound; and
    (c) separating the hydrotalcite-like compound from the suspension.

11. A method according to any one of claims 1 to 3, which comprises:

    (a) reacting a magnesium-containing powder and a transition alumina powder in an aqueous suspension to form a meixnerite intermediate;
    (b) contacting the meixnerite intermediate with an excess of stearate ions; and
    (c) separating the stearate-containing, hydrotalcite-like compound from the suspension.

12. The method of claim 11, wherein the stearate ion contacting step (b) consists essentially of contacting the suspension with stearic acid, a stearic acid salt or combination thereof.

**Patentansprüche**

1. Verfahren zum Herstellen eines blättrigen Doppelhydroxid-Pulvers, welches Verfahren umfaßt:

   (a) Umsetzen von mindestens einem dreiwertigen Metalloxid-Pulver und mindestens einer zweiwertigen Metall-Verbindung, ausgewählt aus der Gruppe, bestehend aus einem Hydroxid, Oxide, Carbonat und Mischungen davon, in einer wässrigen Suspension, um ein Doppelhydroxid-Zwischenprodukt zu erzeugen;
   (b) Kontaktieren des Doppelhydroxid-Zwischenproduktes mit einer Anionen-Quelle, um ein blättriges Doppelhydroxid zu erzeugen; sowie
   (c) Abtrennen des blättrigen Doppelhydroxids aus der Suspension.

**2.** Verfahren nach Anspruch 1, bei welchem (1) das dreiwertige Metalloxid-Pulver eine Verbindung ist, die im wesentlichen aus Übergangs-Aluminiumoxid besteht, und/oder (2) die zweiwertige Metall-Verbindung ausgewählt ist aus der Gruppe, bestehend aus Magnesiumoxid, -hydroxid, -carbonat ' oder Mischungen davon, einer Zinkoxid-enthaltenden Verbindung, einer Kupferoxid-enthaltenden Verbindung, einer Nickeloxid-enthaltenden Verbindung, einer Eisenoxid-enthaltenden Verbindung, einer Calciumoxid-enthaltenden Verbindung oder einer Manganoxid-enthaltenden Verbindung oder Mischungen davon.

**3.** Verfahren nach Anspruch 1 oder 2, bei welchem, (1) das dreiwertige Metalloxid-Pulver im wesentlichen aus einem rehydratisierbaren Aluminiumoxid besteht, vorzugsweise aus einem aktivierten Aluminiumoxid mit einer BET-Oberfläche von etwa 100 $m^2$/g oder darüber, und/oder (2) die zweiwertige Metall-Verbindung ausgewählt ist aus der Gruppe, bestehend aus Magnesiumoxid, -hydroxid, -carbonat oder Mischungen davon, und ist vozugsweise basisches Magnesiumcarbonat, Magnesiumoxid, Hydromagnesit oder Mischungen davon, vozugsweise Hydromagnesit.

**4.** Verfahren nach Anspruch 1, 2 oder 3, bei welchem, die Anionen-Quelle in Schritt (b) ausgewählt ist aus der Gruppe, bestehend aus: Kohlendioxid; einer Carbonat-enthaltenden Verbindung; einer Säure, die ausgewählt ist aus der Gruppe, bestehend aus Bromwasserstoff, Borsäure, Essigsäure, Oxalsäure und Stearinsäure; sowie einem Ammoniumsalz einer Säure, ausgewählt aus der Gruppe, bestehend aus: Bromwasserstoff, Borsäure, Essigsäure, Oxalsäure und Stearinsäure; sowie Mischungen davon.

**5.** Verfahren nach einem der Ansprüche 1 bis 4, welches Verfahren umfaßt:

(a) Umsetzen eines Magnesium-enthaltenden Pulvers und eines Übergangs-Aluminiumoxid-Pulvers in einer wässrigen Suspension, um ein Meixnerit-Zwischenprodukt zu erzeugen;
(b) Kontaktieren des Meixnerit-Zwischenproduktes mit einem Carbonat-enthaltenden Ion, vorzugsweise Kohlendioxid, um eine Hydrotalcit-Verbindung zu erzeugen; sowie
(c) Abtrennen der Hydrotalcit-Verbindung aus der Suspension.

**6.** Verfahren nach einem der Ansprüche 1 bis 3, welches Verfahren umfaßt:

(a) Umsetzen eines Magnesium-enthaltenden Pulvers und eines Übergangs-Aluminiumoxid-Pulvers in einer wässrigen Suspension, um ein Meixnerit-Zwischenprodukt zu erzeugen;
(b) Kontaktieren des Meixnerit-Zwischenproduktes mit einem Alkalimetallsalz, ausgewählt aus der Gruppe, bestehend aus einem Chlorid, einem Bromid oder einem Iodid; oder einer Mischung davon; sowie
(c) Abtrennen der Hydrotalcit-ähnlichen Verbindung aus der Suspension.

**7.** Verfahren nach Anspruch 6, bei welchem das Alkalimetallsalz Natriumbromid ist, um in Schritt (c) eine bromierte, Hydrotalcit-ähnliche Verbindung zu erzeugen; oder Natriumchlorid ist, um in Schritt (c) eine chlorierte, Hydrotalcit-ähnliche Verbindung zu erzeugen; und/oder Schritt (a) wird ausgeführt bei einer oder mehreren Temperaturen oberhalb von 80 °C, vorzugsweise 98 ° ... 150 °C.

**8.** Verfahren nach einem der Ansprüche 1 bis 3, welches Verfahren umfaßt:

(a) Umsetzen eines Magnesium-enthaltenden Pulvers und eines Übergangs-Aluminiumoxid-Pulvers in einer wässrigen Suspension, um ein Meixnerit-Zwischenprodukt zu erzeugen;
(b) Kontaktieren des Meixnerit-Zwischenproduktes mit einem Überschuß von Borat-Ionen, um eine Bor-enthaltende, Hydrotalcit-ähnliche Verbindung zu erzeugen; sowie
(c) Abtrennen der Bor-enthaltenden, Hydrotalcit-ähnlichen Verbindung aus der Suspension.

**9.** Verfahren nach Anspruch 8, bei welchem der Schritt (b) zum Kontaktieren mit Borat-Ionen das Zusetzen einer Borverbindung zu der Suspension umfaßt, welche Verbindung ausgewählt ist aus der Gruppe, bestehend aus $H_3BO_3$, $H_3B_3O_6$, $Na_2B_2O_7 \cdot 10H_2O$, $Na_2B_2O_7$, $NaBO_2NaB_5O_8$ oder $K_2B_{10}O_{16}$; oder Mischungen davon.

**10.** Verfahren nach einem der Ansprüche 1 bis 3, welches Verfahren umfaßt:

(a) Umsetzen eines Magnesium-enthaltenden Pulvers und eines Übergangs-Aluminiumoxid-Pulvers in einer wässrigen Suspension, um ein Meixnerit-Zwischenprodukt zu erzeugen;
(b) Kontaktieren des Meixnerit-Zwischenproduktes mit einem Überschuß von Oxalat-Ionen, um eine Oxalat-enthaltende, Hydrotalcit-ähnliche Verbindung zu erzeugen; sowie
(c) Abtrennen der Oxalat-enthaltenden, Hydrotalcitähnlichen Verbindung aus der Suspension.

**11.** Verfahren nach einem der Ansprüche 1 bis 3, welches Verfahren umfaßt:

(a) Umsetzen eines Magnesium-enthaltenden Pulvers und eines Übergangs-Aluminiumoxid-Pulvers in einer wässrigen Suspension, um ein Meixnerit-Zwischenprodukt zu erzeugen;

(b) Kontaktieren des Meixnerit-Zwischenproduktes mit einem Überschuß von Stearat-Ionen, um eine Stearat-enthaltende, Hydrotalcit-ähnliche Verbindung zu erzeugen; sowie

(c) Abtrennen der Stearat-enthaltenden, Hydrotalcitähnlichen Verbindung aus der Suspension.

**12.** Verfahren nach Anspruch 11, bei welchem der Schritt (b) des Kontaktierens mit Stearat-Ionen im wesentlichen besteht aus dem Kontaktieren der Suspension mit Stearinsäure, einem Salz der Stearinsäure oder einer Kombination davon.

## Revendications

**1.** Procédé pour préparer une poudre d'hydroxyde double stratifié comprenant :

(a) la réaction d'au moins une poudre d'oxyde métallique trivalent et d'au moins un composé métallique divalent choisi dans le groupe constitué par un hydroxyde, un oxyde, un carbonate et leurs mélanges, dans une suspension aqueuse pour former un intermédiaire hydroxyde double ;

(b) la mise en contact de l'intermédiaire hydroxyde double avec une source d'anions pour former un hydroxyde double stratifié ; et

(c) la séparation de l'hydroxyde double stratifié à partir de la suspension.

**2.** Procédé selon la revendication 1, dans lequel (1) la poudre d'oxyde métallique trivalent est un composé constitué essentiellement d'alumine de transition ; et/ou (2) le composé métallique divalent est choisi dans le groupe constitué par un oxyde, hydroxyde ou carbonate de magnésium, ou un de leurs mélanges, un composé contenant de l'oxyde de zinc, un composé contenant de l'oxyde de cuivre, un composé contenant de l'oxyde de nickel, un composé contenant de l'oxyde de fer, un composé contenant de l'oxyde de calcium, ou un composé contenant de l'oxyde de manganèse, ou un de leurs mélanges.

**3.** Procédé selon la revendication 1 ou 2, dans lequel (1) la poudre d'oxyde métallique trivalent est constituée essentiellement d'une poudre d'alumine pouvant être réhydratée, de préférence une alumine activée ayant une aire spécifique BET d'environ 100 $m^2/g$ ou plus ; et/ou (2) le composé métallique divalent est choisi dans le groupe constitué par un oxyde, hydroxyde ou carbonate de magnésium, ou un de leurs mélanges, et de préférence est du carbonate de magnésium basique, de l'oxyde de magnésium, de l'hydromagnésite ou un de leurs mélanges, de préférence l'hydromagnésite.

**4.** Procédé selon la revendication 1, 2 ou 3, dans lequel la source d'anions dans l'étape (b) est choisie dans le groupe constitué par : le dioxyde de carbone ; un composé contenant un carbonate ; un acide choisi dans le groupe constitué par : l'acide bromhydrique, l'acide borique, l'acide acétique, l'acide oxalique, l'acide stéarique ; et un sel d'ammonium d'un acide choisi dans le groupe constitué par : l'acide bromhydrique, l'acide borique, l'acide acétique, l'acide oxalique, l'acide stéarique et leurs mélanges.

**5.** Procédé selon l'une quelconque des revendications 1 à 4, qui comprend :

(a) la réaction d'une poudre contenant du magnésium et d'une poudre d'alumine de transition dans une suspension aqueuse pour former un intermédiaire meixnérite ;

(b) la mise en contact de l'intermédiaire meixnérite avec un ion contenant un carbonate, de préférence le dioxyde de carbone, pour former un composé hydrotalcite ; et

(c) la séparation du composé hydrotalcite à partir de la suspension.

**6.** Procédé selon l'une quelconque des revendications 1 à 3, qui comprend :

(a) la réaction d'une poudre contenant du magnésium et d'une poudre d'alumine de transition dans une suspension aqueuse pour former un intermédiaire meixnérite ;

(b) la mise en contact de l'intermédiaire meixnérite avec un sel de métal alcalin choisi dans le groupe constitué par un chlorure, un bromure ou un iodure ou un de leurs mélanges ; et

(c) la séparation du composé de type hydrotalcite à partir de la suspension.

**7.** Procédé selon la revendication 6, dans lequel le sel de métal alcalin est du bromure de sodium pour produire un composé de type hydrotalcite bromé dans l'étape (c) ou est du chlorure de sodium pour produire un composé de type hydrotalcite chloré dans l'étape (c) ; et/ou l'étape (a) est réalisée à une ou plusieurs températures au-delà de 80°C, de préférence de 98 à 150°C.

**8.** Procédé selon l'une quelconque des revendications 1 à 3, qui comprend :

(a) la réaction d'une poudre contenant du magnésium et d'une poudre d'alumine de transition dans une suspension aqueuse pour former un intermédiaire meixnérite ;

(b) la mise en contact de l'intermédiaire meixnérite avec un excès d'ions borate pour former un composé de type hydrotalcite contenant du bore ; et

(c) la séparation du composé de type hydrotalcite contenant du bore à partir de la suspension.

9. Procédé selon la revendication 8, dans lequel l'étape (b) de mise en contact des ions borate comprend l'addition à la suspension d'un composé du bore qui est choisi dans le groupe constitué par $H_3BO_3$, $H_3B_3O_6$, $Na_2B_7O_7 \cdot 10H_2O$, $N_2B_2O_7$, $NaBO_2NaB_5O_8$, ou $K_2B_{10}O_{16}$ ou leurs mélanges.

10. Procédé selon l'une quelconque des revendications 1 à 3, qui comprend :

(a) la réaction d'une poudre contenant du magnésium et d'une poudre d'alumine de transition dans une suspension aqueuse pour former un intermédiaire meixnérite ;

(b) la mise en contact de l'intermédiaire meixnérite avec un excès d'ions oxalate pour former un composé de type hydrotalcite contenant de l'oxalate ; et

(c) la séparation du composé de type hydrotalcite à partir de la suspension.

11. Procédé selon l'une quelconque des revendications 1 à 3, qui comprend :

(a) la réaction d'une poudre contenant du magnésium et d'une poudre d'alumine de transition dans une suspension aqueuse pour former un intermédiaire meixnérite ;

(b) la mise en contact de l'intermédiaire meixnérite avec un excès d'ions stéarate ; et

(c) la séparation du composé de type hydrotalcite contenant du stéarate à partir de la suspension.

12. Procédé selon la revendication 11, dans lequel l'étape (b) de mise en contact des ions stéarate est constituée essentiellement de la mise en contact de la suspension avec de l'acide stéarique, un sel d'acide stéarique ou une de leurs combinaisons.